# EUROPEAN PATENT SPECIFICATION

(11) **EP 4 299 096 B1**
(45) Date of publication and mention of the grant of the patent: **28.01.2026**
(21) Application number: 22795968.1
(22) Date of filing: 23.03.2022
(51) Int. Cl.: A61M 16/12, A61M 16/10, A61M 11/00, A61M 15/00, A61M 16/16, A61M 16/00, A61M 16/08

(54) **DRUG AEROSOL SUPPLY APPARATUS FOR RESPIRATOR**
ARZNEIMITTELAEROSOLVERSORGUNGSVORRICHTUNG FÜR BEATMUNGSGERÄT
APPAREIL D'ALIMENTATION EN AÉROSOL MÉDICAMENTEUX POUR RESPIRATEUR

(30) Priority: 26.04.2021 KR 20210053453
(43) Date of publication of application: 03.01.2024
(73) Proprietor: Boditech Med Inc., Chuncheon-si, Gangwon-do 24398 (KR)
(72) Inventor: CHOI, Doo Young, Gwangmyeong-si Gyeonggi-do 14318 (KR)
(74) Representative: Ter Meer Steinmeister & Partner
(86) International application number: PCT/KR2022/004025
(87) International publication number: WO 2022/231134

(56) References cited:
- WO-A1-2009/042187
- WO-A1-2009/135871
- WO-A1-2013/163527
- WO-A1-2016/151029
- WO-A1-2019/236896
- WO-A1-2019/236899
- WO-A1-2020/236860
- WO-A2-2005/102431
- WO-A2-2007/008825
- GB-A- 2 543 906
- KR-A- 20070 004 058
- KR-A- 20100 060 004
- KR-A- 20200 141 437
- KR-B1- 102 105 887
- KR-B1- 102 228 450
- US-A- 5 237 987
- US-A- 5 388 571
- US-A- 5 762 063
- US-A1- 2005 211 245
- YANG SSU-HAN ET AL: "Size distribution of salbutamol/ipratropium aerosols produced by different nebulizers in the absence and presence of heat and humidification", PULMORNARY PHARMACOLOGY & THERAPEUTICS, ACADEMIC PRESS, GB, vol. 48, 18 October 2017 (2017-10-18), pages 22 - 27, XP085412854, ISSN: 1094-5539, DOI: 10.1016/J.PUPT.2017.10.009

## Description

### Technical Field

The present disclosure relates to an aerosol drug supply apparatus for a respirator, the aerosol drug supply apparatus being configured such that a drug in an aerosol state is included in air that is conditioned in the respirator, and the aerosol drug supply apparatus being configured such that the drug is capable of being transmitted to a patient's lungs through the patient's respiratory tract when the patient inhales the air.

### Background Art

Generally, a patient who has difficulty in spontaneous breathing relies on a respirator to breathe. In addition, a drug may be prescribed according to a decision of a doctor, and the drug may be included in air conditioned by a respirator and then provided to a patient. Various drugs may be used according to a patient's physical condition and, for example, a drug that helps a lung function may be used when a patient's lung function is bad.

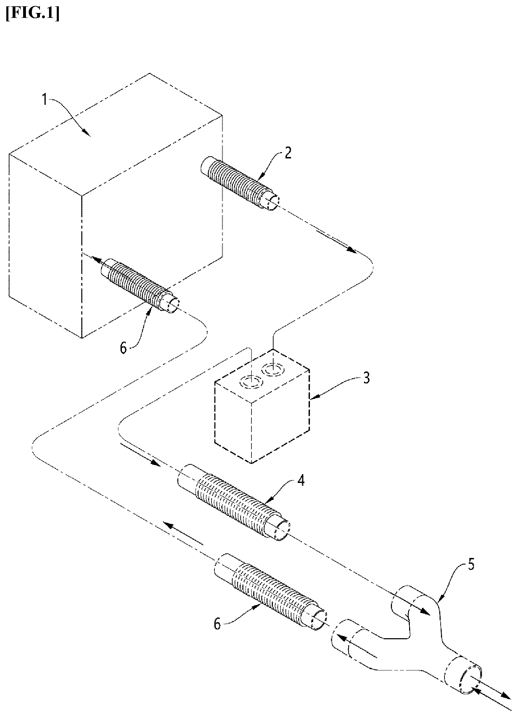

A configuration of a typical respirator will be described with reference to FIG. 1. FIG. 1 is a view illustrating a configuration of a respirator.

A typical respirator includes an air purifier 1, a first tube 2, a heating and humidifying apparatus 3, a second tube 4, a Y-tube 5, and a return tube 6.

The air purifier 1 is configured to purify air, to adjust an oxygen concentration appropriately, and to set a pressure and a speed.

First air is conditioned and discharged from the air purifier 1, and the first air reaches the heating and humidifying apparatus 3 along the first tube 2.

The heating and humidifying apparatus 3 is configured to increase a humidity of the first air and to increase a temperature of the first air by heating the first air. Generally, the temperature may be set to a temperature similar to body temperature, for example, 36 degrees Celsius to 37 degrees Celsius.

Second air conditioned in the heating and humidifying apparatus 3 reaches the Y-tube 5 along the second tube 4.

In the Y-tube 5, an inlet into which the second air is introduced, an entrance connected to a mask or a mouthpiece, and an outlet through which exhaled air is discharged are formed.

In the Y-tube 5, an air flow may be changed according to an inhalation breath and an exhalation breath. For example, when a patient breathes in, the second air may flow to the entrance through the inlet. When the patient breathes out, an air flow from the entrance to the outlet is formed in the Y-tube 5.

The outlet of the Y-tube 5 is connected to the return tube 6, the return tube 6 is connected to the air purifier 1, and the air purifier 1 is configured to condition air of the exhalation breath.

Meanwhile, a drug may be prescribed according to a condition of a patient, and in order to administer the drug, generally, a T-tube is inserted between the heating and humidifying apparatus 3 and the second tube 4, and the drug is injected into the T-tube.

The T-tube is capable of converting a state of a drug into an aerosol state in a jet method. In the jet method, when the speed of an air flow is fast, pressure is lowered and a drug is suctioned by the low pressure, and then the drug is changed into a fine particle state by a jet stream.

However, the jet method conventionally used may change physical properties of the conditioned first air or the conditioned second air. In order to use the jet method, highpressure air is required to be additionally supplied from the outside, there is a problem that an oxygen concentration may be reduced in the supply process, and there is a problem that a pressure of air may be increased or a flow speed of the air may be increased.

Particularly, even though an oxygen concentration is set according to a lung function of a patient, an oxygen concentration of air actually provided to the patient is low, so that a serious problem may occur due to an oxygen deficiency phenomenon.

Meanwhile, when a pressure of air provided to a patient is high or a providing speed of air is high, there are problems that the patient's breathing becomes uncomfortable and the patient cannot breathe naturally.

Meanwhile, conventionally, a drug is injected into the T-tube, and the drug in an aerosol state is included in the T-tube as the second air passes, so that there is a problem that the drug is continuously consumed regardless of a breathing state of a patient. Particularly, when the patient exhales, there is a problem that the drug is wasted since the drug is discharged immediately rather than reaching the patient's lungs.

### [Documents of Related Art]

(Patent Document 1) KR 10-1301163 B1
(Patent Document 2) KR 10-2021-0018306 A
(Patent Document 3) KR 10-2020-0026879 A
(Patent Document 4) WO 2019/236896 A1 discloses a breath-enhanced nebulizer adapted for use in administering a nebulized pharmaceutical composition to a patient by inhalation wherein the patient is connected to a mechanical ventilator.
(Patent Document 5) WO 2005/102431 A2 discloses a ventilator system including a ventilator in fluid communication with an inspiratory tube an and expiratory tube which converge at junction device, and a patient interface device in fluid communication with the junction device. Further, the system includes an ultrasonic nebulizer attached to and in fluid communication with the junction deviceor or with inspiratory tube. During operation of ventilator system, a pressurized flow of gas is introduced into inspiratory tube from ventilator and passes to and through junction device, wherein the nebulizer emits an aerosolized medicament into gas flow to produce combined gas flow containing aerosol particles of medicament. Gas flow is transported through junction device to patient interface device.
(Patent Document 6) WO 2007/008825 A2 discloses a system for delivery of an aerosolized formulation to the respiratory tract of a ventilated host, comprising a ventilator, an inspiration sensor in communication with an inspiratory pathway of the ventilator and operative to detect an initiation of respiration, an aerosol generator operative to aerosolize and release a formulation to be delivered to the respiratory tract of the host, and an electronic controller that controls the timing of aerosol generation such that aerosols are only generated during select intervals of the respiratory cycle.
(Patent Document 7) WO 2009/042187 A1 discloses a system to introduce aerosolized medicament to a patient, the system comprising a humidifier coupled to an inspiratory limb of a ventilator circuit wye, wherein the humidifier supplies heated and humidified air to the patient, an endotracheal tube having a proximal end coupled to a distal end of the ventilator circuit wye, and an ultrasonic nebulizer coupled to the endotracheal tube, wherein the nebulizer generates the aerosolized medicament from a medicament source supplied to the nebulizer. The system comprises a breathing characteristics sensor which sends breathing characteristic information to the a controller to allow the controller to select an appropriate delivery cycle of the aerosolized liquid to the patient.
Document WO2019/236899 A1 discloses a ventilator circuit apparatus for the administration of nebulized drugs through an endotracheal tube to a patient on a mechanical ventilator that controls breathing gases to the patient, comprising a breathing circuit with an inspiratory limb and exhalation limb connected to the ventilator; an ultrasonic nebulizer on the inspiratory limb interposed between a T-fitting and a three-way valve such that the nebulizer can be removed from the inspiratory limb without interrupting the flow of breathing gases to the patient, and a humidifier or heat and moisture exchanger on the inspiratory limb interposed between the nebulizer and the endotracheal tube.

### Disclosure

### Technical Problem

Accordingly, the present disclosure has been made keeping in mind the above problems occurring in the related art, and an objective of the present disclosure is to provide an aerosol drug supply apparatus for a respirator, the aerosol drug supply apparatus being configured such that when a drug is administered to a patient relying on the respirator to assist the patient's lung function, a drug is accurately sprayed when the patient inhales air, and the aerosol drug supply apparatus being configured to maintain physical properties such as oxygen concentration, pressure, speed, and so on of both first air and second air that are conditioned.

### Technical Solution

The invention is defined in the appended independent claims. In order to achieve the above objectives, according to an embodiment of the present disclosure, there is provided an aerosol drug supply apparatus for a respirator, the aerosol drug supply apparatus including: an air purifier 1 configured to discharge first air in which an oxygen concentration, a pressure, and a speed thereof are set; an inhaled air sensor 10 connected to the air purifier 1, the inhaled air sensor 10 being configured to sense the first air and to generate a sensing signal; a first tube 2 which is connected to the inhaled air sensor 10 and along which the first air moves; a heating and humidifying apparatus 3 connected to the first tube 2, the heating and humidifying apparatus 3 being configured to generate second air by adjusting a temperature and a humidity of the first air, and the heating and humidifying apparatus 3 being configured to discharge the second air; a second tube 4 which is connected to the heating and humidifying apparatus 3 and along which the second air moves; a T-tube 20 having a straight tube 22 that has one side thereof provided with a branch tube 24, the straight tube 22 being connected to the second tube 4, thereby allowing the second air to pass therethrough; a drug fine particle generator 30 mounted on the branch tube 24, the drug fine particle generator 30 being configured to be operated only when the sensing signal is input, and the drug fine particle generator 30 being configured to convert a state of a drug into a fine particle state by using ultrasonic vibration and to spray a drug in a fine particle state to the second air; a Y-tube 5 having one side connected to the T-tube 20, the Y-tube 5 having another side connected to a mask or a mouthpiece; and a return tube 6 having one side connected to the Y-tube 5, the return tube (6) having another side connected to the air purifier 1, thereby allowing exhaled air to flow therethrough.

In addition, in the aerosol drug supply apparatus for the respirator according to the technology (but outside the claimed invention), the sensing signal may be replaced with one of signals including: 1) a signal generated when a chest sensor mounted on a patient's chest detects that the patient's chest expands; 2) a specific brain wave signal generated when the patient is breathing; 3) a signal generated by detecting an air flow of inhaled air in the mask or the mouthpiece; 4) a signal generated by detecting an air pressure of the inhaled air in the mask or the mouthpiece; and 5) a signal generated by detecting an air temperature of the inhaled air in the mask or the mouthpiece.

In addition, in the aerosol drug supply apparatus for the respirator according to an embodiment of the present disclosure, the drug fine particle generator 30 may include: an insertion tube 32 assembled to the branch tube 24; a fine particle generating element 34 disposed on an upper portion of the insertion tube 32 and configured to convert a state of the drug into the fine particle state; a medication case 36 which is disposed on an upper portion of the fine particle generating element 34 and in which the drug is stored; a cover 40 covering the medication case 36; and a packing closure 50 which is mounted on the cover 40 and in which a packing 52 having elasticity is disposed, and the drug may be injected into the medication case 36 by piercing the packing 52 with a syringe needle.

Details of other embodiments are included in the detailed description and drawings.

### Advantageous Effects

In the aerosol drug supply apparatus for the respirator according to the present disclosure, the drug fine particle generator is disposed at an inlet of the Y-tube, and is configured to convert the state of the drug into the fine particle state by using ultrasonic vibration, so that a distance from a position where the drug exists in an aerosol state to the mask or the mouthpiece may be configured as short as possible. Furthermore, the oxygen concentration, the pressure, and the speed of both the first air and the second air conditioned in the air purifier and the heating and humidifying apparatus may be maintained, so that a patient can breathe more naturally.

In addition, in the aerosol drug supply apparatus for the respirator according to an embodiment of the present disclosure, since the drug fine particle generator is operated according to a signal generated from the inhaled air sensor, the drug may be sprayed at an accurate time when the drug is required to be consumed, thereby being capable of significantly reducing drug waste.

In addition, in the aerosol drug supply apparatus for the respirator according to an embodiment of the present disclosure, an exposure of an inner portion of the medication case may be minimized or the exposure of the inner portion of the medication case may be fundamentally prevented, so that the medication case is capable of being prevented from being contaminated. Furthermore, a patient can live in a cleaner environment.

### Description of Drawings

FIG. 1 is a view illustrating a configuration of a respirator.
FIG. 2 is a view illustrating an aerosol drug supply apparatus for a respirator according to an embodiment of the present disclosure.
FIG. 3 is a view illustrating main configurations of the aerosol drug supply apparatus for the respirator according to an embodiment of the present disclosure.

### Best Mode

The attached drawings for illustrating exemplary embodiments of the present disclosure are referred to in order to gain a sufficient understanding of the present disclosure, the merits thereof, and the objectives accomplished by the implementation of the present disclosure.

Hereinafter, embodiments of the present disclosure will be described in detail with reference to the accompanying drawings. It should be understood that the embodiments described below are illustratively shown to aid understanding of the present disclosure, and that the present disclosure may be implemented with various modifications different from the embodiments described herein. However, when it is determined that a detailed description of a related well-known function or component may unnecessarily obscure the gist of the present disclosure while describing the present disclosure, the detailed description and specific illustration thereof will be omitted. In addition, the accompanying drawings may not be drawn to scale, but the size of some components may be exaggerated to aid in understanding of the present disclosure.

Terms such as 'first' and 'second' are used herein merely to describe a variety of constituent elements, but the constituent elements are not limited by the terms. The terms are used only for the purpose of distinguishing one constituent element from another constituent element. For example, a first element may be termed a second element, and a second element may be termed a first element, without departing from the scope of the present disclosure.

The terms used in the specification are defined in consideration of functions used in the present disclosure, and can be changed according to the intent or conventionally used methods of producers. Accordingly, definitions of the terms should be understood on the basis of the entire description of the present specification.

Like reference numerals refer to like elements throughout the specification.

### [Description of Reference Numerals]

1: air purifier 2, 4, 6: first, second, and third tubes
3: heating and humidifying apparatus 5: Y-tube 10: inhaled air sensor 20: T-tube
22: straight tube 24: branch tube
30: drug fine particle generator 32: insertion tube
34: fine particle generating element 36: medication case
40: cover 50: packing closure
52: packing

### Mode for Invention

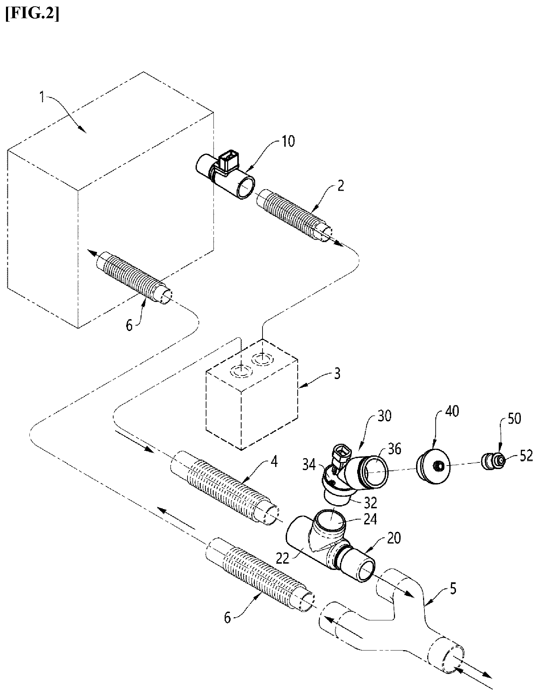

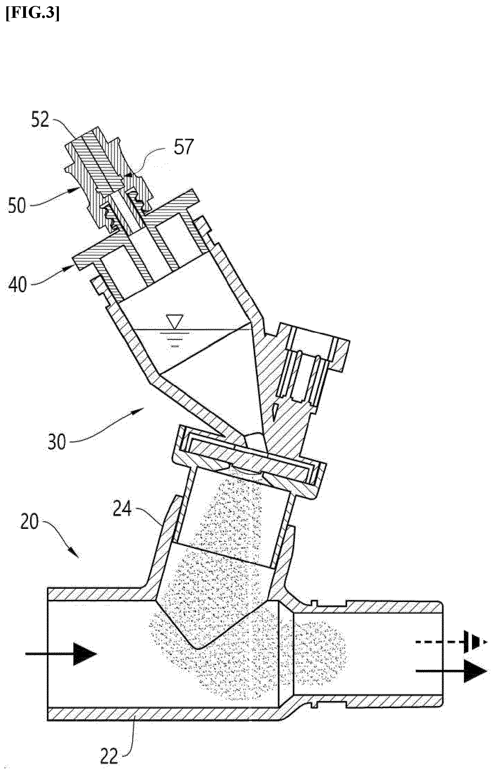

Hereinafter, referring to FIG. 2 and FIG. 3, an aerosol drug supply apparatus for a respirator according to an embodiment of the present disclosure will be described. FIG. 2 is a view illustrating an aerosol drug supply apparatus for a respirator according to an embodiment of the present disclosure. FIG. 3 is a view illustrating main configurations of the aerosol drug supply apparatus for the respirator according to an embodiment of the present disclosure.

An aerosol drug supply apparatus for a respirator according to an embodiment of the present disclosure may include an inhaled air sensor 10, a T-tube 20, a cover 40, and a packing closure 50.

An air purifier 1 is configured to discharge a first air in which an oxygen concentration, a pressure, and a speed thereof are set. The oxygen concentration may be set according to a capability of a patient's lung function. For example, when a capability of a patient's lung function is half that of a normal person's lung function, the oxygen concentration may be set to approximately 40%.

The inhaled air sensor 10 is connected to the air purifier 1, and is configured to sense the first air and to generate a sensing signal when the first air is discharged from the inhaled air sensor 10.

A first tube 2 is connected to the inhaled air sensor 10, and the first air moves along the first tube 2.

A heating and humidifying apparatus 3 is connected to the first tube 2, is capable of generating a second air by adjusting a temperature and a humidity of the first air, and is configured to discharge the second air.

A second tube 4 is connected to the heating and humidifying apparatus 3, and the second air moves along the second tube 4.

The T-tube 20 has a straight tube 22 having one side thereof provided with a branch tube 24, and the straight tube 22 is connected to the second tube 4, so that the second air is capable of passing through the T-tube 20.

A drug fine particle generator 30 is mounted on the branch tube 24, and is operated only when the sensing signal is input, so that a drug in a fine particle state may be sprayed to the second air.

A Y-tube 5 may have one side connected to the T-tube 20, and may have another side connected to a mask or a mouthpiece.

A return tube 6 has one side connected to the Y-tube 5 and has another side connected to the air purifier 1, thereby allowing exhaled air to flow along the return tube.

In the aerosol drug supply apparatus for the respirator according to the present disclosure, the drug fine particle generator 30 is disposed at an inlet of the Y-tube 5, and is configured to convert a state of a drug into a fine particle state by using ultrasonic vibration, so that a distance from a position where the drug exists in an aerosol state to a mask or a mouthpiece may be configured as short as possible. That is, a path in which the drug in the fine particle state moves is very short, so that a drug effect may be expected to be good even when a small amount of the drug is used and changed into the fine particle state.

In addition, in the aerosol drug supply apparatus for the respirator according to an embodiment of the present disclosure, the oxygen concentration, the pressure, and the speed of both the first air and the second air conditioned in the air purifier and the heating and humidifying apparatus may be maintained by preventing additional air input from the outside, thereby being capable of allowing a patient to breathe more naturally.

In addition, in the aerosol drug supply apparatus for the respirator according to an embodiment of the present disclosure, since the drug fine particle generator is operated according to a signal generated from the inhaled air sensor, a drug may be sprayed at an accurate time when the drug is required to be consumed, thereby being capable of significantly reducing drug waste.

In the aerosol drug supply apparatus for the respirator according to an embodiment of the present disclosure, the sensing signal for operating the drug fine particle generator 30 may vary as described below, in addition to sensing the first air discharged from the air purifier 1.
1) The sensing signal may be replaced with a signal generated when a chest sensor mounted on a patient's chest detects that the patient's chest expands. When the patient breathes, the chest rises and falls repeatedly, and such movements may be detected and used as the sensing signal.
2) The sensing signal may be a specific brain wave signal generated when the patient is breathing. A specific brain wave is generated in the brain when lungs are operated, and the specific brain wave may be detected and used as the sensing signal.
3) The sensing signal may be replaced with a signal generated by detecting an air flow of inhaled air in a mask or a mouthpiece. There is a difference in a direction of the air flow between inhaled air and exhaled air, and such a difference may be detected and used as the sensing signal.
4) The sensing signal may be replaced with a signal generated by detecting an air pressure of inhaled air in a mask or a mouthpiece. There is a difference in a pressure between inhaled air and exhaled air, and such a difference may be detected and used as the sensing signal.
5) The sensing signal may be replaced with a signal generated by detecting an air temperature of inhaled air in a mask or a mouthpiece. There is a difference in a temperature between inhaled air and exhaled air, and such a difference may be detected and used as the sensing signal.

That is, in the aerosol drug supply apparatus for the respirator according to an embodiment of the present disclosure, the sensing signal generated in the inhaled air sensor 10 may be replaced (outside the current invention) with one of the various examples described above, or all of the various examples described above may be applied so that the drug fine particle generator may be generated when any one of the sensing signals is generated.

As illustrated in FIG. 3, the drug fine particle generator 30 may include an insertion tube 32, a fine particle generating element 34, a medication case 36, the cover 40, and the packing closure 50.

The insertion tube 32 may be assembled to the branch tube 24.

The fine particle generating element 34 may be disposed on an upper portion of the insertion tube 32, and is configured to convert a state of a drug into a fine particle state. For example, the fine particle generating element 34 may change a form of a drug to a fine particle form, and such a technology is realized by using a known technology, so that a more detailed description will be omitted.

The medication case 36 is disposed on an upper portion of the fine particle generating element 34, and a drug is stored in the medication case 36.

The cover 40 covers the medication case 36 so that the drug stored in the medication case 36 is not exposed to the outside.

The packing closure 50 may be mounted on the cover 40, and a packing 52 having elasticity may be disposed in the packing closure 50. More specifically, the packing 52 may be formed of soft rubber. Accordingly, when a syringe needle pierces the packing 52, the syringe needle may penetrate the packing 52. Furthermore, when the syringe needle is pulled out, a piercing made by the syringe needle is immediately filled by the elasticity of the packing 52. Therefore, the medication case 36 may be separated from the outside at all times.

That is, in the aerosol drug supply apparatus for the respirator according to an embodiment of the present disclosure, a drug may be injected into the medication case 36 by piercing the packing 52 with the syringe needle.

Therefore, in the aerosol drug supply apparatus for the respirator according to an embodiment of the present disclosure, an inner portion of the medication case 36 may be fundamentally blocked from being exposed, so that contamination of the medication case 36 is prevented. Furthermore, a patient can live in a cleaner environment.

In addition, by preventing the exposure of the inner portion of the medication case 36, an entire circuit section of the respirator, especially an entire section of the tube connected from the air purifier 1 to the mask or the mouthpiece, may be strictly prevented from being exposed to the outside.

Although an exemplary embodiment of the present disclosure was described above with reference to the accompanying drawings, those skilled in the art would understand that the present disclosure may be implemented in various ways without changing the necessary features of the prevent disclosure.

Therefore, it should be understood that the embodiment described above is not limitative, but only an example in all respects, the scope of the present disclosure is expressed by claims described below, not the detailed description, and it should be construed that all of changes and modifications achieved from the meanings and scope of claims which follow.

### Industrial Applicability

An aerosol drug supply apparatus for a respirator according to an embodiment of the present disclosure may be used for including a drug in conditioned air when the conditioned air conditioned from the respirator is provided to a patient.

## Claims

1. A aerosol drug supply apparatus for a respirator, the aerosol drug supply apparatus comprising:
an air purifier (1) configured to discharge first air in which an oxygen concentration, a pressure, and a speed thereof are set;
an inhaled air sensor (10) connected to the air purifier (1), the inhaled air sensor (10) being configured to sense the first air and to generate a sensing signal;
a first tube (2) which is connected to the inhaled air sensor (10) and along which the first air moves;
a heating and humidifying apparatus (3) connected to the first tube (2), the heating and humidifying apparatus (3) being configured to generate second air by adjusting a temperature and a humidity of the first air, and the heating and humidifying apparatus (3) being configured to discharge the second air;
a second tube (4) which is connected to the heating and humidifying apparatus (3) and along which the second air moves;
a T-tube (20) having a straight tube (22) that has one side thereof provided with a branch tube (24), the straight tube (22) being connected to the second tube (4), thereby allowing the second air to pass therethrough;
a drug fine particle generator (30) mounted on the branch tube (24), the drug fine particle generator (30) being configured to be operated only when the sensing signal is input, and the drug fine particle generator (30) being configured to convert a state of a drug into a fine particle state by using ultrasonic vibration and to spray the drug in the fine particle state to the second air;
a Y-tube (5) having one side connected to the T-tube (20), the Y-tube (5) having another side connected to a mask or a mouthpiece; and
a return tube (6) having one side connected to the Y-tube (5), the return tube (6) having another side connected to the air purifier (1), thereby allowing exhaled air to flow therethrough,
wherein the drug fine particle generator (30) is disposed at an inlet of the Y-tube (5), and
wherein the drug fine particulate generator (30) is operated according to the signal generated from the inhaled air sensor (10).

2. The aerosol drug supply apparatus of claim 1, wherein the drug fine particle generator (30) comprises:
an insertion tube (32) assembled to the branch tube (24);
a fine particle generating element (34) disposed on an upper portion of the insertion tube (32) and configured to convert a state of the drug into the fine particle state;
a medication case (36) which is disposed on an upper portion of the fine particle generating element (34) and in which the drug is stored;
a cover (40) covering the medication case (36); and
a packing closure (50) which is mounted on the cover (40) and in which a packing (52) having elasticity is disposed, and
the drug is injected into the medication case (36) by piercing the packing (52) with a syringe needle.

## Patentansprüche

1. Aerosol-Arzneimittelzuführvorrichtung für einen Respirator, wobei die Aerosol-Arzneimittelzuführvorrichtung Folgendes umfasst:
einen Luftreiniger (1), der konfiguriert ist, erste Luft abzugeben, bei der die Sauerstoffkonzentration, der Druck und die Geschwindigkeit festgelegt sind;
einen Sensor (10) für inhalierte Luft, der mit dem Luftreiniger (1) verbunden ist, wobei der Sensor (10) für inhalierte Luft konfiguriert ist, die erste Luft zu messen und ein Messsignal zu erzeugen;
ein erstes Rohr (2), das mit dem Sensor (10) für inhalierte Luft verbunden ist und längs dessen sich die erste Luft bewegt;
eine Heiz- und Befeuchtungsvorrichtung (3), die mit dem ersten Rohr (2) verbunden ist, wobei die Heiz- und Befeuchtungsvorrichtung (3) konfiguriert ist, zweite Luft zu erzeugen, indem die Temperatur und die Feuchtigkeit der ersten Luft angepasst werden, und wobei die Heiz- und Befeuchtungsvorrichtung (3) konfiguriert ist, die zweite Luft abzugeben;
ein zweites Rohr (4), das mit der Heiz- und Befeuchtungsvorrichtung (3) verbunden ist und längs dessen sich die zweite Luft bewegt;
ein T-Rohr (20), das ein gerades Rohr (22) aufweist, wovon eine Seite mit einem verzweigten Rohr (24) versehen ist, wobei das gerade Rohr (22) mit dem zweiten Rohr (4) verbunden ist, so dass die zweite Luft dort hindurch gelangen kann;
einen Generator (30) für Arzneimittel-Feinpartikel, der am verzweigten Rohr (24) montiert ist, wobei der Generator (30) für Arzneimittel-Feinpartikel so konfiguriert ist, dass er nur dann betrieben wird, wenn das Messsignal eingegeben wird, und wobei der Generator (30) für Arzneimittel-Feinpartikel konfiguriert ist, einen Zustand eines Arzneimittels durch Verwenden von Ultraschallschwingung in einen Feinpartikelzustand zu überführen und das Arzneimittel im Feinpartikelzustand zur zweiten Luft zu sprühen;
ein Y-Rohr (5), wovon eine Seite mit dem T-Rohr (20) verbunden ist, wobei das Y-Rohr (5) eine weitere Seite hat, die mit einer Maske oder einem Mundstück verbunden ist; und
ein Rückführrohr (6), wovon eine Seite mit dem Y-Rohr (5) verbunden ist, wobei das Rückführrohr (6) eine weitere Seite hat, die mit dem Luftreiniger (1) verbunden ist, so dass ausgeatmete Luft dort hindurchströmen kann,
wobei der Generator (30) für Arzneimittel-Feinpartikel am Einlass des Y-Rohrs (5) angeordnet ist, und
wobei der Generator (30) für Arzneimittel-Feinpartikel entsprechend dem Signal betrieben wird, das vom Sensor (10) für inhalierte Luft erzeugt wird.

2. Aerosol-Arzneimittelzuführvorrichtung nach Anspruch 1, wobei der Generator (30) für Arzneimittel-Feinpartikel Folgendes umfasst:
ein Einsetzrohr (32), das mit dem Verzweigungsrohr (24) zusammengebaut wird;
ein Element (34) zum Erzeugen von Feinpartikeln, das an einem oberen Abschnitt des Einsetzrohrs (32) angeordnet ist und konfiguriert ist, einen Zustand des Arzneimittels in den Feinpartikelzustand zu überführen;
ein Medikationsgehäuse (36), das an einem oberen Abschnitt des Elements (34) zum Erzeugen von Feinpartikeln angeordnet ist und in dem das Arzneimittel gespeichert ist;
eine Abdeckung (40), die das Medikationsgehäuse (36) bedeckt; und
einen Verpackungsverschluss (50), der an der Abdeckung (40) montiert ist, und in dem eine Verpackung (52), die eine Elastizität aufweist, angeordnet ist, und
wobei das Arzneimittel in das Medikationsgehäuse (36) mittels Durchstechen der Verpackung (52) mit einer Spritzennadel eingespritzt wird.

## Revendications

1. Appareil d'alimentation en médicament en aérosol pour un respirateur, l'appareil d'alimentation en médicament en aérosol comportant :
un purificateur d'air (1) configuré pour évacuer un premier air dont une concentration d'oxygène, une pression et une vitesse sont réglées ;
un capteur d'air inhalé (10) relié au purificateur d'air (1), le capteur d'air inhalé (10) étant configuré pour détecter le premier air et pour générer un signal de détection ;
un premier tube (2) qui est relié au capteur d'air inhalé (10) et le long duquel le premier air se déplace ;
un appareil de chauffage et d'humidification (3) relié au premier tube (2), l'appareil de chauffage et d'humidification (3) étant configuré pour générer un second air en ajustant une température et une humidité du premier air, et l'appareil de chauffage et d'humidification (3) étant configuré pour évacuer le second air ;
un second tube (4) qui est relié à l'appareil de chauffage et d'humidification (3) et le long duquel le second air se déplace ;
un tube en T (20) ayant un tube droit (22) dont un côté est pourvu d'un tube d'embranchement (24), le tube droit (22) étant relié au second tube (4), en permettant ainsi au second air de passer à travers celui-ci ;
un générateur de fines particules de médicament (30) monté sur le tube d'embranchement (24), le générateur de fines particules de médicament (30) étant configuré pour fonctionner uniquement lorsque le signal de détection est entré, et le générateur de fines particules de médicament (30) étant configuré pour convertir un état d'un médicament en un état de fines particules en utilisant des vibrations ultrasonores et pour pulvériser le médicament à l'état de fines particules dans le second air ;
un tube en Y (5) ayant un côté relié au tube en T (20), le tube en Y (5) ayant un autre côté relié à un masque ou à un embout buccal ; et
un tube de retour (6) ayant un côté relié au tube en Y (5), le tube de retour (6) ayant l'autre côté relié au purificateur d'air (1), en permettant ainsi à l'air exhalé de s'écouler à travers celui-ci,
dans lequel le générateur de fines particules de médicament (30) est disposé à une entrée du tube en Y (5), et
dans lequel le générateur de fines particules de médicament (30) fonctionne en fonction du signal généré à partir du capteur d'air inhalé (10).

2. Appareil d'alimentation en aérosol de médicament selon la revendication 1, dans lequel le générateur de fines particules de médicament (30) comporte :
un tube d'insertion (32) assemblé au tube d'embranchement (24) ;
un élément générateur de fines particules (34) disposé sur une partie supérieure du tube d'insertion (32) et configuré pour convertir un état du médicament en état de fines particules ;
un boîtier de médicament (36) qui est disposé sur une partie supérieure de l'élément générateur de fines particules (34) et dans lequel le médicament est stocké ;
un couvercle (40) recouvrant le boîtier de médicament (36) ; et
une fermeture de garniture (50) qui est montée sur le couvercle (40) et dans laquelle est disposée une garniture (52) ayant une élasticité, et
le médicament est injecté dans le boîtier de médicament (36) en perçant la garniture (52) avec une aiguille de seringue.
